# EUROPEAN PATENT SPECIFICATION

(11) **EP 1 485 715 B1**
(45) Date of publication and mention of the grant of the patent: **11.04.2007**
(21) Application number: 03744496.5
(22) Date of filing: 14.02.2003
(51) Int. Cl.: G01N 33/50

(54) **SEROLOGICAL METHOD FOR DETECTING INTESTINAL MICROVILLI ATROPHY IN PATIENTS SUFFERING FROM COELIAC DISEASE**
SEROLOGISCHE METHODE ZUM NACHWEIS VON DARMZOTTEN-ATROPHIE BEI ZÖLIAKIE/SPRUE-PATIENTEN
METHODE SEROLOGIQUE DE DETECTION D'UNE ATROPHIE MICROVILLOSITAIRE DE L'INTESTIN CHEZ DES PATIENTS ATTEINTS DE MALADIE COELIAQUE

(30) Priority: 15.03.2002 IT RM20020144
(43) Date of publication of application: 15.12.2004
(73) Proprietor: Dipartimento di Scienze Biomediche e Biotecnologie, 09121 Cagliari (IT)
(72) Inventor: DE VIRGILIIS, Stefano, 09121 Cagliari (IT); CLEMENTE, Maria Grazia, 09121 Cagliari (IT)
(74) Representative: Capasso, Olga
(86) International application number: PCT/IT2003/000080
(87) International publication number: WO 2003/079012

(56) References cited:
- WO-A-97/10508
- CLEMENTE M G ET AL: "IMMUNE REACTION AGAINST THE CYTOSKELETON IN COELIAC DISEASE" GUT, BRITISH MEDICAL ASSOCIATION, LONDON,, GB, vol. 47, no. 4, October 2000 (2000-10), pages 520-526, XP009011811 ISSN: 0017-5749 cited in the application
- CANCADO EDUARDO LUIZ R ET AL: "Heat serum inactivation as a mandatory procedure for antiactin antibody detection in cell culture." HEPATOLOGY, vol. 23, no. 5, 1996, pages 1098-1104, XP009016962 ISSN: 0270-9139

## Description

The present invention relates to a serological method for detecting intestinal microvilli atrophy in patients suffering from coeliac disease.

More specifically the invention relates to a method for detecting intestinal microvilli atrophy in patients suffering from coeliac disease by detection of anti-actin antibodies in the patient serum.

Diagnosis of coeliac disease or permanent gluten intolerance is based on gluten-dependency demonstration of clinic symptoms, circulating antibodies and histological lesions of bowel mucosa. Diagnostic method currently includes carrying out of at least a bowel biopsy, invasive instrumental test which often requires sedation or general anaesthesia (Revised criteria for diagnosis of coeliac disease. Report of Working Group of European Society of Paediatric Gastroenterology and Nutrition. Arch Dis Child 1990; 65:909-11).

Current serological methodologies for the determination of anti-gluten, anti-endomysium and anti-transglutaminase antibodies which demonstrate the presence of an autoimmune reaction in susceptible subjects, allow only to diagnostically suspect the coeliac disease and suggest to carry out an intestinal biopsy to confirm the diagnosis.

It is noteworthy to point out that the diagnosis expression and abstinence and dietetic therapy prescription can be carried out only following detection of intestinal lesions.

Further in serologically positive subjects intestinal biopsy often is difficult to be understood exactly because it shows variable situations in comparison to normal histology (potential coeliac disease), light and not specific inflammatory signs with normal intestinal microvilli and variable intestinal microvilli atrophy degrees.

In addition lesions cannot be uniformly distributed within intestinal mucosa whereby it is possible a misunderstanding depending on mucosa areas wherein bioptic sampling was carried out.

From recent studies of the authors of the invention (Clemente MG, Musu MP, Frau F, Busco G, Sole G, Corazza GR, De Virgiliis S. Immune reaction against the cytoskeleton in coeliac disease. Gut 2000; 47(4): 520-6) the presence of serum anti-F-actin antibodies in patients suffering from coeliac disease has been detected. The presence of these antibodies in sera has been demonstrated to be strictly related to the occurrence of intestinal microvilli atrophy in patients suffering from coeliac disease. However described methodology is not sufficiently sensitive since about 20 % of patients suffering from intestinal atrophy are not found out resulting in potential diagnosis mistakes.

Nowadays a non invasive test suitable to reveal the presence of intestinal mucosa alterations due to coeliac disease and being sufficiently sensitive and therefore reliable is not yet available as alternative to intestinal biopsy.

In view of the above it is therefore clear the need to provide more sensitive methods for serological diagnosis of intestinal microvilli atrophy in coeliac disease occurrence which, in addition to avoid an invasive instrumental test, allow also possible diagnostic mistakes to be reduced.

The authors of the present invention now provide a method for diagnosis of intestinal microvilli atrophy in patients suffering from coeliac disease in active phase by detection of anti-actin antibodies in sera from said patients which meets the above reported requirements allowing the identification in 100 % of the cases.

Method is based on the detection in the peripheral blood of IgA type circulating autoantibodies against F-actin which represents the main protein of cellular cytoskeleton. The presence of these autoantibodies in sera from coeliac patients is related to the presence of moderate or severe villous atrophy in intestinal mucosa whereby an important diagnostic tool which allows intestinal biopsy to be avoided is provided.

It has been observed that anti-F actin antibodies are no more detected after the recovery from lesions of intestinal mucosa following discontinuation from gluten containing food ingestion. Therefore the method is advantageously used to study the effects of diet or therapeutic actions in a patient.

The detection of anti-actin antibodies according to the present invention uses indirect immunofluorescence analysis carried out on epithelial cell culture from rat small bowel. Those skilled in the art will recognise that also cells from other animal species are within the scope of the invention.

Cells are grown on microscopy slides in the presence of colchicine to promote F-actin intracellular increase. Then the cells are fixed and made permeable. Thus prepared slides are incubated with sera sample to be tested. The presence of anti F-actin antibodies in the patient sera is detected by using IgA type anti- immunoglobulin anti-serum conjugated with fluorescein isothiocyanate. Evaluation of the results is carried out using fluorescence optic microscope. Alternative detection methods are as well within the scope of the invention.

The method for detecting anti F-actin antibodies according to the invention can be advantageously used for coeliac disease clinically suspected and specific antibodies positive patients (anti-gluten, anti-endomysium or anti-transglutaminase) and can avoid intestinal biopsy in all positive patients to be carried out.

It is therefore an object of the present invention a method for detecting intestinal microvilli atrophy in a patient suffering from coeliac disease including immunological detection of ant -F-actin autoantibodies in a biological fluid from said patient consisting of the following steps:
a) preparation of a normal epithelial cell culture from small bowel of an experiment animal;
b) incubation with the patient fluid;
c) detection of the binding of the anti-F-actin autoantibodies to the cells.

Further the method according to the invention can include, following step a), the incubation with a substance able to induce the polymerisation of globular actin to filaments such as colchicine.

The culture of small bowel epithelial cells suitable to be used consists of rat cell culture. Preferably epithelial cells of IEC-6 lineage are used.

The method for detecting binding of anti F-actin antibodies to the cells can include the addition of IgA type anti-immuno globulin anti-serum conjugated with a fluorochrome such as fluorescein isothiocyanate and the detection of fluorescence signal, for example by means of fluorescence microscopy.

Factors allowing the inventive method to be more sensitive than known ones result from the use of intestinal epithelial cells (IEC-6) which are of the same cellular type as those causing *in vivo* the production of anti F-actin autoantibodies in coeliac patient and the induction of higher amount of intracellular actin by means of the addition of colchicine. In fact the latter, increasing the amount of intracellular actin filaments, also causes the increase of the amount of antigen recognised by autoantibodies allowing to detect the presence of autoatibodies also in sera with lower titer thereof.

The present invention will now be described, by way of illustration but not limitation, using the below reported examples.

Example 1: Comparison between the sensitivity of the method according to the invention and the method based on the use of laryngeal carcinoma epithelial cell (HEP-2) (Clemente MG, Musu MP, Frau F, Busco G, Sole G, Corazza GR, De Virgiliis S. Immune reaction against the cytoskeleton in coeliac disease. Gut 2000; 47(4): 520-6).

One hundred and two coeliac patients (age range: 1-60 years) serologically positive for anti-endomysium, anti-transglutaminase and anti-gluten antibodies were studied according to both of the methods. When intestinal biopsies were carried out 17 patients did not show at all intestinal microvilli atrophy while the remaining 85 showed a moderate or severe degree thereof according to Table 1

**Table 1**

| | **HEp-2** cell method | **IEC-6** cell method |
|---|---|---|
| Total | 102 | 102 |
| Age (range, years) | 1-60 | 1-60 |
| IgA anti-endomysium positive | 102 | 102 |
| IgA anti-transglutaminase positive | 102 | 102 |
| Intestinal microvilli atrophy | 85 | 85 |
| Absence of intestinal microvilli atrophy | 17 | 17 |
| Serum IgA anti F-actin positive | 69 | 85 |
| Serum IgA anti F-actin negative | 33 | 17 |
| **F-actin positive with atrophy** | **69** | **85** |
| F-actin positive without atrophy | 0 | 0 |
| **F-actin negative with atrophy** | **16** | **0** |
| F-actin negative without atrophy | 17 | 17 |

### Materials and Methods

### Culture of IEC-6 cells

Rat small bowel epithelial cells (IEC-6, ATCC n. CRL-1592, American Type Collection, Manassas, VA, USA) were grown in cell culture flasks (Falcon Labware) at 37°C in 95 % air and 5 % CO₂ atmosphere. Growth culture medium was Dulbecco modified Eagle medium (D-MEM, American Type Collection, Manassas, VA, USA) containing 4,500 mg/L D-glucose, pyridoxine hydrochloride, heat inactivated (56°C, 30 minutes) 5 % fetal bovine serum (FBS), 0,1 U/ml bovine insulin, 4 mM L-glutamine, 50 U/ml penicillin, and 50 µg/ml streptomycin.

### IEC-6 cell culture on multiple well slides in the presence of colchicine:

When growing cells are at a 80-90 % confluence they are washed in saline (phosphate buffer solution, PBS) and gently scraped off culturing flask by treatment over 2-3 minutes with a solution containing 0,25 % trypsin, 1 mM EDTA (Gibco brl). After re-suspension in culture medium the cells (2 x 10⁴ cell/ml) are seeded in multispot slide wells (ICN). When growing cells are at a 30-50 % confluence colchicine to a 0,1 mM final concentration is added to medium culture over a two hour incubation period.

### Indirect immunofluorescence for determination of anti F-actin antibodies in the patient serum:

Slides are washed twice in PBS and covered with fixing solution containing 2 % paraformaldehyde in PBS (1 g paraformaldehyde added to 50 ml PBS under continuous stirring until dissolution with further addition of a NaOH solid "drop", pH adjusted to 7,4-7,6 with H₃P0₄) for 30 minutes at room temperature. After washing in PBS slides are covered for 15 minutes at room temperature with PBS permeabilising solution containing 0,5 % TritonX-100. Following three PBS washings, test sera, previously inactivated at 56°C for 30 minutes and centrifuged (20000 rcf) for 10 minutes, are added to the wells at a 1 :5 dilution in PBS. Sera are incubated for 1 hour at room temperature and in moist chamber. After 3 washings in PBS anti-IgA human fluorescein isothiocyanate conjugated antiserum (MEDIC), diluted 1:100 in PBS and centrifuged (20000 rcf) for 10 minutes, is added to the wells. Antiserum is incubated for one hour at room temperature in moist chamber in the dark. Then the slides are washed twice in alone PBS and once again in the presence of evans blue for 10 minutes After two further washings in PBS covering slides are mounted using a 50 % glycerol solution in PBS and results are analysed using a fluorescence microscope (Olympus).

Hep-2 control cells are used as described in Clemente MG, Musu MP, Frau F, Busco G, Sole G, Corazza GR, De Virgiliis S. Immune reaction against the cytoskeleton in coeliac disease. Gut 2000; 47(4): 520-6.

### Evaluation of Results:

In the presence of anti F-actin antibodies, actin filament bundles are detected by fluorescence microscopy due to their fluorescent coloration. Both intracellular filaments, which are seen as parallel bundles extending from one to the other cellular pole, and as microvilli constituents of cellular membrane appearing as radial extroversions on the outer side of cells.

### Inventive method using IEC-6 cells

Eighty five of 102 (83 %) coeliac studied patients were positive for IgA anti F-actin. All these patients had a moderate or severe histological degree of intestinal microvilli atrophy. None (0 %) of 17 patients without intestinal microvilli atrophy was positive for anti F-actin antibodies.

### Method using HEp-2 cells

Sixty nine of 102 coeliac observed patients (68 %) were positive. Positive patients had a moderate or severe histological degree of intestinal microvilli atrophy. Thirty three patients were negative for anti-actin antibodies. Therefore by using this method sixteen patients were not revealed (48,8 % of negative patients).

Statistical difference among the results obtained using two above methods is very significant. Test-χ², p = 0,0007.

Sensitivity values of above described methods with reference to the presence of histological lesions from moderate or severe atrophy in coeliac subjects are as below:
- 100 % for the method according to the invention using IEC-6 cells and addition of colchicine;
- 81 % for the known method using Hep-2 cells.

## Claims

1. Method for detecting intestinal microvilli atrophy in a patient suffering from coeliac disease including immunological detection of anti-F-actin autoantibodies in a biological fluid from said patient consisting of the following steps:
a) preparation of a normal epithelial cell culture from small bowel of an experiment animal;
b) incubation with the patient fluid;
c) detection of the binding of the anti-F-actin autoantibodies to the cells.

2. Method according to claim 1 further including, following step a), the incubation with a substance able to induce the polymerisation of globular actin to filaments.

3. Method according to claim 2 wherein the substance able do induce the polymerisation of globular actin to filaments is colchicine.

4. Method according to claim 1 wherein the culture of small bowel epithelial cells is from rat.

5. Method according to claim 1 wherein the culture of small bowel epithelial cells is from IEC-6 cell line.

6. Method according to claim 1 wherein the method for detecting binding of anti F-actin autoantibodies to the cells includes the addition of IgA type anti-immuno globulin anti-serum conjugated with fluorochrome and detection of fluorescence signal.

7. Method according to claim 6 wherein the fluorochrome is fluorescein isotiocyanate.

## Patentansprüche

1. Verfahren zum Nachweisen einer Atrophie der Mikrovilli im Darm bei einem Patienten, der an Zöliakie leidet, einschließlich des immunologischen Nachweises von Anti-F-Aktin-Antikörper in einer biologischen Flüssigkeit aus dem Patienten, wobei das Verfahren aus den folgenden Schritten besteht:
a) Herstellung einer Kultur aus normalen Epithelzellen aus dem Dünndarm eines Versuchstieres;
b) Inkubation mit der Patientenflüssigkeit;
c) Nachweis der Bindung der Anti-F-Aktin-Antikörper an die Zellen.

2. Verfahren nach Anspruch 1, das des Weiteren nach Schritt a) die Inkubation mit einer Substanz umfasst, die in der Lage ist, die Polymerisation von globulärem Aktin zu Filamenten zu induzieren.

3. Verfahren nach Anspruch 2, wobei es sich bei der Substanz, die in der Lage ist, die Polymerisation von globulärem Aktin zu Filamenten zu induzieren, um Colchizin handelt.

4. Verfahren nach Anspruch 1, wobei die Kultur von Epithelzellen aus dem Dünndarm aus der Ratte stammt.

5. Verfahren nach Anspruch 1, wobei die Kultur von Epithelzellen aus dem Dünndarm aus einer IEC-6-Zelllinie stammt.

6. Verfahren nach Anspruch 1, wobei das Verfahren zum Nachweisen der Bindung von Anti-F-Aktin-Antikörper an die Zellen die Zugabe von Anti-Immunglobulin-Antiserum vom IgA-Typ, konjugiert mit Fluorochrom, und den Nachweis des Fluoreszenzsignals umfasst.

7. Verfahren nach Anspruch 6, wobei es sich bei dem Fluorochrom um Fluoreszeinisothiocyanat handelt.

## Revendications

1. Dispositif destiné à détecter une atrophie microvillositaire intestinale chez un patient souffrant d'une maladie coeliaque, comprenant la détection immunologique d'auto-anticorps anti-actine F dans un fluide biologique dudit patient, comprenant les étapes suivantes :
a) préparation d'une culture de cellules épithéliales normales provenant de l'intestin grêle d'un animal de laboratoire ;
b) incubation avec le fluide du patient ;
c) détection de la liaison des auto-anticorps anti-actine F avec les cellules.

2. Dispositif selon la revendication 1, comprenant en outre, après l'étape a) l'incubation avec une substance capable d'induire la polymérisation de l'actine globulaire en filaments.

3. Dispositif selon la revendication 2, dans laquelle la substance capable d'induire la polymérisation de l'actine globulaire en filaments est la colchicine.

4. Dispositif selon la revendication 1, dans lequel la culture de cellules épithéliales de l'intestin grêle provient d'un rat.

5. Dispositif selon la revendication 1, dans lequel la culture de cellules épithéliales de l'intestin grêle provient de cellules IEC-6.

6. Dispositif selon la revendication 1, dans lequel la méthode de détection de la liaison des auto-anticorps anti-actine F avec les cellules comprend l'ajout d'un anti-sérum anti-immunoglobulines de type IgA conjugué à un fluorochrome et à la détection du signal fluorescent.

7. Dispositif selon la revendication 6, dans lequel le fluorochrome est l'isothiocyanate de fluorescéine.
